(19) Europäisches Patentamt
European Patent Office
Office européen des brevets

(11) Veröffentlichungsnummer : **0 504 791 B1**

# (12) EUROPÄISCHE PATENTSCHRIFT

(45) Veröffentlichungstag der Patentschrift :
**26.01.94 Patentblatt 94/04**

(51) Int. Cl.$^5$ : **A61K 7/09,** A61K 7/06

(21) Anmeldenummer : **92104567.0**

(22) Anmeldetag : **17.03.92**

## (54) Mittel zur Verformung von menschlichen Haaren und Verwendung von Ammoniumcarbamat und solchen Mitteln.

(30) Priorität : **22.03.91 DE 4109365**

(43) Veröffentlichungstag der Anmeldung :
**23.09.92 Patentblatt 92/39**

(45) Bekanntmachung des Hinweises auf die Patenterteilung :
**26.01.94 Patentblatt 94/04**

(84) Benannte Vertragsstaaten :
**AT BE CH DE DK ES FR GB IT LI NL SE**

(56) Entgegenhaltungen :
**EP-A- 0 320 612**
**EP-A- 0 363 057**
**US-A- 2 708 940**

(73) Patentinhaber : **GOLDWELL AKTIENGESELLSCHAFT**
**Zerninstrasse 10-18**
**D-64297 Darmstadt (DE)**

(72) Erfinder : **Rose, Burkhard**
**Katharinenstrasse 14**
**W-6100 Darmstadt 13 (DE)**
Erfinder : **Tennigkeit, Jürgen, Dr.**
**Felsbergstrasse 51**
**W-6104 Seeheim 2 (DE)**

EP 0 504 791 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Mittel zur dauerhaften Verformung von menschlichen Haaren, d.h., ein Dauerwellmittel, das verbesserte Gebrauchseigenschaften aufweist.

Bekanntlich erfordert die Dauerwellung zwei Behandlungsschritte:

Die reduktive Spaltung der Cystin-Disulfidbrücken des Haares durch Einwirkung eines Reduktionsmittels und die anschließende Fixierung durch Aufbringung eines Oxidationsmittels, wodurch die Cystin-Disulfidbrücken wieder hergestellt werden.

Das klassische Reduktionsmittel ist, wie bereits aus den Pionierpatenten DE-PS 948 186 und 972 424 hervorgeht, dabei die Thioglykolsäure, z.B. als Ammonium- oder Monoethanolaminsalz, die in den letzten Jahren durch Glycerinmonothioglykolat teilweise ersetzt wurde; jedoch werden auch Thiomilchsäure und deren Ester sowie auch anorganische Sulfite eingesetzt.

Die Thioglykolat enthaltenden Zusammensetzungen weisen dabei einen pH-Wert im Bereich zwischen etwa 7,5 und etwa 9,0, inbesondere 8,5 bis 9,0, auf, wobei die Alkalisierung heute in der Regel, neben Ammoniak, durch Zusatz von Ammonium-(bi)-carbonat erzielt wird. (vgl. beispielsweise US-PS 2,708,940).

Die Reduktionittel-Zusammensetzungen enthalten häufig auch etwa 0,5 bis etwa 35 Gew.-%, vorzugsweise etwa 1, insbesondere etwa 2,5 bis etwa 15 Gew.-% Alkandiole bzw. deren Ether, die auch als Lösungs- und Penetrationsmittel oder Lösungsvermittler bzw. Carrier dienen können.

Diese Zusammensetzungen sind insbesondere hinsichtlich ihrer Wellwirksamkeit jedoch nach wie vor verbesserungsfähig.

Es wurde nun gefunden, daß man die Verformungsfähigkeit von Dauerwellmitteln, d.h., deren Reduktionsphase, die $C_3$-$C_6$-Alkandiole und/oder deren Ether enthält, dadurch verbessern und eine Wirksamkeitssteigerung erreichen kann, wenn man ihnen als Alkalisierungsmittel Ammoniumcarbamat,

$$NH_4\text{-}O\text{-}\underset{\underset{O}{\|}}{C}\text{-}NH_2 ,$$

in einer Menge von etwa 0,5 bis etwa 10 Gew.-%, vorzugsweise 2 bis 8 Gew.-%, insbesondere 3 bis 6 Gew.-%, bezogen auf die Gesamtzusammensetzung der Reduktionsphase, zusetzt.

Darüber hinaus hat sich gezeigt, daß durch die erfindungsgemäße Kombination auch der Geruch bei der Anwendung verbessert wird. Erfindungsgemäß kann das Ammoniumcarbamat alleine oder auch im Gemisch mit untergeordneten Anteilen bekannter Alkalisierungsmittel zum Einsatz gelangen, beispielsweise Ammoniak und Ammoniumcarbonat bzw. -bicarbonat und Monoethanolamin.

Die Reduktionsmittel werden in Form von wässrigen Lösungen, Gelen, Emulsionen (Cremes) oder auch als Aerosolschäume eingesetzt und enthalten, neben dem Reduktions- und Alkalisierungsmittel, haarkonditionierende Substanzen, beispielsweise kationische Polymere, gegebenenfalls Verdickungsmittel, sogenannte Carrier, die insbesondere auch die Penetration des Produktes erhöhen, Komplexbildner, Trübungsmittel, Duftstoffe und, zur Verbesserung des Netz- und Penetrationsvermögens, auch oberflächenaktive Substanzen.

Wesentlicher Bestandteil der Fixier- bzw. Neutralisationsmittel sind Oxidationsmittel. Das am häufigsten benutzte Oxidationsmittel ist Wasserstoffperoxid, dessen Konzentration im Fixierungsmittel üblicherweise zwischen etwa 0,5 und etwa 3 Gew.-% liegt.

Weitere zum Einsatz gelangende Oxidationsmittel sind Alkalibromate, Harnstoffperoxid und Natriumperborat, die beiden letztgenannten selbstverständlich in wasserfreien Produkten.

Diese Zusammensetzungen werden vorzugsweise in Form wässriger Lösungen oder als Aerosolschäume, seltener als Gele, konfektioniert.

Diese Zusammensetzungen enthalten verschiedene weitere Bestandteile wie Stabilisatoren, Pflanzenextrakte, haarkonditionierende Wirkstoffe und Tenside.

Als reduzierende Wirkstoffe in den erfindungsgemäßen Dauerwellmitteln werden, wie bereits erwähnt, neben anorganischen Sulfiten wie Natriumbisulfit, insbesondere Thioglykolsäure und Ammoniumthioglykolat, Thiomilchsäure, deren Salze und Ester, Cystein und dessen Hydrochlorid, Cysteamin, N-Acetylcystein, Thioessigsäure, deren Salze und Ester sowie insbesondere Thioglykolsäuremonoglycerinester eingesetzt. In Falle von dessen Verwendung oder der Verwendung ähnlicher Thioester erfolgt die Vermischung mit der restlichen Reduktionsmittel-Zusammensetzung unmittelbar vor der Applikation.

Die Anwendungskonzentration des reduzierenden Wirkstoffs liegt in Abhängigkeit von der Struktur desselben im allgemeinen zwischen etwa 1 und etwa 15 Gew.-% der Reduktionsmittel-Zusammensetzung, vorzugsweise zwischen etwa 3 und etwa 10 Gew.-%.

Die Menge an Alkalisierungsmittel ist abhängig vom reduzierenden Wirkstoff. Vorzugsweise enthält die

Reduktionsmittel-Zusammensetzung etwa 1 bis etwa 10, insbesondere etwa 2 bis etwa 8 Gew.-% Ammoniumcarbamat.

Der Gehalt an Ammoniumcarbamat ist natürlich auch davon abhängig, ob und wieviel weitere Alkalisierungsmittel wie Ammoniak und/oder Ammonium(bi)carbonat zugesetzt werden. Es wird die Einstellung eines pH-Wertes im Bereich zwischen etwa 7 und etwa 9 angestrebt.

Die erfindungsgemäßen Dauerwellmittel enthalten vorzugsweise auch Tenside. Deren Anteil liegt bei 0,1 bis etwa 10, insbesondere etwa 1 bis etwa 5 Gew.-%.

Sowohl bei den in den Redüktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C8-C18-Fettalkoholpolyglykolether, Fettsäurepolyglykolester, Fettsäurealkanolamide, Aminoxide und vor allem C8-C18-Alkylpolyglykoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Der weitere obligatorische Bestandteil der erfindungsgemäßen Zusammensetzungen ist ein $C_3$-$C_6$-Alkandiol bzw. dessen Ether, insbesondere Mono-$C_1$-$C_3$-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen etwa 1 und etwa 30, vorzugsweise etwa 2,5 bis etwa 15, insbesondere etwa 5 bis etwa 10 Gew.-% der Reduktionsmittel-Zusammensetzung.

Neben den $C_3$-$C_6$-Alkandiolen bzw. deren Ethern können zusätzlich auch Propylencarbonat (4-Methyl-1,3-dioxolan-2-on), N-Alkylpyrrolidone, Glycerin und Harnstoff Verwendung finden.

Die erfindungsgemäßen Mittel können selbstverständlich alle in Dauerwellmitteln üblichen Stoffe enthalten, auf deren detaillierte Aufzählung hier verzichtet wird.

Zur Vermeidung von Wiederholungen wird vielmehr auf den Stand der Technik verwiesen, wie er beispielsweise in "Ullmann's Encyclopedia of Industrial Chemistry", Vol. A12 (1986), S.588 bis 591, sowie insbesondere der Monographie von K. Schrader. "Grundlagen und Rezepturen der Kosmetika", 2. Aufl. (1989, Hüthig-Verlag), S.823 bis 840 sowie in dem Übersichtsartikel von Dr. Hollenberg et al in "Seifen-Öle-Fette-Wachse" 117 (1991), S.81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Die folgenden Ausführungsbeispiele dienen der näheren Illustration der Erfindung.

## BEISPIEL 1

### a) Reduktionslösung

| | | |
|---|---|---|
| Thioglykolsäure, 80-%ig | | 13,0 (Gew.-%) |
| Ammoniumcarbamat | | 7,0 |
| Ammoniak | | 1,7 |
| $C_{12}$-$C_{14}$-Alkylpolyglykosid (Kondensationsgrad 1,8) | | 2,0 |
| 1,4-Butandiol | | 3,5 |
| Parfümöl, Trübungsmittel | | q.s. |
| Wasser | ad | 100,0 |

b) Fixierlösung

| | | | |
|---|---|---|---|
| Wasserstoffperoxid | | 2,2 | (Gew.-%) |
| Kationisches Polymer (z.B. Polymer JR) | | 0,2 | |
| $C_{12}$-$C_{14}$-Fettalkoholpolyglykolether | | 1,0 | |
| Parfümöl, Stabilisator | | q.s. | |
| Wasser | ad | 100,0 | |

BEISPIEL 2

a) Reduktionslösung

| | | | |
|---|---|---|---|
| Thioglykolsäure, 80-%ig | | 10,0 | (Gew.-%) |
| Ammoniumcarbamat | | 6,0 | |
| Ammoniak | | 1,1 | |
| Cocoamidopropylbetain | | 2,2 | |
| 1,2-Propandiol | | 5,0 | |
| Parfümöl | | q.s. | |
| Wasser | ad | 100,0 | |

b) Fixierlösung

| | | | |
|---|---|---|---|
| Wasserstoffperoxid | | 2,3 | (Gew.-%) |
| Pentaoxyethylstearylammoniumchlorid | | 1,3 | |
| $C_9$-$C_{11}$-Alkylpolyglycosid (Kondensationsgrad 1,4) | | 2,3 | |
| Citronensäure | | 0,2 | |
| Parfümöl, Acetanilid | | q.s. | |
| Wasser | ad | 100,0 | |

BEISPIEL 3

a) Reduktionsmittel

| | | | |
|---|---|---|---|
| Thiomilchsäure | | 12,5 | (Gew.-%) |
| Ammoniak, 25-%ig | | 5,0 | |
| Ammoniumcarbamat | | 7,6 | |
| Harnstoff | | 4,0 | |
| Quaternäres kationisches Polymeres | | 0,5 | |
| Nichtionischer Fettsäurepolyglykolester | | 2,3 | |
| Cocobetain | | 0,8 | |
| Dipropylenglykolmonomethylether | | 3,5 | |
| Trübungsmittel, Parfümöl, Pflanzenextrakt | | q.s. | |
| Wasser | ad | 100,0 | |

b) Fixiermittel

| | | |
|---|---|---|
| Wasserstoffperoxid, 50-%ig | | 4,8 (Gew.-%) |
| Citronensäure | | 0,4 |
| Kamillenextrakt | | 0,3 |
| $Na_2HPO_4$ | | 0,4 |
| Natriumpolyglykolethersulfat | | 2,5 |
| Parfümöl, Stabilisator, Trübungsmittel | | q.s. |
| Wasser | ad | 100,0 |
| Phosphorsäure zur Einstellung auf pH 3 | | |

BEISPIEL 4

a) Reduktionsmittel

| | | |
|---|---|---|
| Thioglykolsäure, 80-%ig | | 11,5 (Gew.-%) |
| Ammoniumcarbamat | | 4,5 |
| Ammoniumcarbonat | | 2,0 |
| $C_8$-$C_{12}$-Alkylpolyglykosid (2,0) | | 1,0 |
| Ricinusöloxethylat (40 E0) | | 1,0 |
| 1-Methoxypropanol(-2) | | 2,5 |
| Parfümöl | | q.s. |
| Wasser | ad | 100,0 |

b) Fixiermittel

| | | |
|---|---|---|
| Natriumbromat | | 12,5 (Gew.-%) |
| Harnstoff | | 5,2 |
| Lauryldimethylaminoxid | | 1,8 |
| Parfümöl, Farbstoff, Pflanzenextrakt | | q.s. |
| Wasser | ad | 100,0 |

BEISPIEL 5

a) Reduktionsmittel

| | | |
|---|---|---|
| Ammoniumthioglykolat | | 5,0 (Gew.-%) |
| Cystein | | 2,5 |
| Ammoniumcarbamat | | 3,8 |
| 1-Methoxypropanol(-2) | | 5,2 |
| 1,3-Butandiol | | 4,8 |
| Oleylpolyglykolether | | 3,0 |
| Oleylpolyglykosid (Kondensationsgrad 1,8) | | 0,2 |
| Parfümöl | | q.s. |
| Wasser | ad | 100,0 |

**b) Fixiermittel**

| | | |
|---|---|---|
| Wasserstoffperoxid | | 2,5 (Gew.-%) |
| Cetylstearylalkohol | | 2,0 |
| Natriumlaurylethersulfat | | 1,2 |
| $C_{12}$-$C_{18}$-Alkylpolyglykolether | | 1,0 |
| Stabilisator, Parfümöl | | q.s. |
| Wasser | ad | 100,0 |

**BEISPIEL 6**

1 Teil einer

Zusammensetzung A aus
60 Gew.-% Glycerinmonothioglykolsäureester, 80%ig (Rest Glycerin),
15 Gew.-% Glycerinmono-2-thiopropionsäureester, 80%ig (Rest Glycerin)
25 Gew.-% 1,2-Propandiol/Dipropylenglykol (Gewichtsverhältnis 2 : 1)
wird mit 2 Teilen einer bis zur Anwendung davon getrennt gehaltenen Zusammensetzung B aus
1,0 Gew.-% Ammoniumcarbamat,
0,3 Gew.-% Eiweißhydrolysat,
1,5 Gew.-% $C_9$-$C_{11}$-Alkylpolyglykosid (1,3)
0,5 Gew.-% Ricinusölpolyglykolfettsäureester
0,4 Gew.-% Parfümöl
10,0 Gew.-% 1,2-Propandiol
82,3 Gew.-% Wasser,
mit Ammoniak auf pH 7 eingestellt, vermischt, auf das Haar aufgebracht und 10 bis 30 Minuten einwirken gelassen.

Nach dem Spülen mit Wasser wird mit einer Zusammensetzung C fixiert.

**Zusammensetzung C:**

| | | |
|---|---|---|
| Wasserstoffperoxid | | 2,40 (Gew.-%) |
| Kationisches Polymer | | 0,50 |
| Citronensäure | | 0,20 |
| Laurylpolyglycolether | | 2,00 |
| $Na_2HPO_4$ | | 0,20 |
| Phosphorsäure | | 0,15 |
| Stabilisator | | q.s. |
| Wasser | ad | 100,00 |

Es wird ein glänzendes dauergewelltes Haar ohne jede Reizung der Kopfhaut erhalten.

## BEISPIEL 7

### a) Reduktionslösung

| | | |
|---|---|---|
| Thiomilchsäure | | 12,5 (Gew.-%) |
| Ammoniak | | 1,5 |
| Ammoniumcarbamat | | 5,0 |
| Harnstoff | | 4,3 |
| Kationisches Polymeres | | |
| (z.B. Polydimethyldiallylammoniumchlorid) | | 0,5 |
| Nichtionisches Tensid | | 1,0 |
| (Fettalkoholpolyglykolether, | | |
| (Fettsäureamidoalkylbetain) | | |
| Dipropylenglykolmonomethylether | | 5,0 |
| 1,2-Propandiol | | 5,0 |
| Parfümöl | | q.s. |
| Wasser | ad | 100,00 |

### b) Fixiermittel

| | | |
|---|---|---|
| Wasserstoffperoxid | | 2,4 (Gew.-%) |
| Lauryldimethylaminoxid | | 2,0 |
| Citronensäure | | 0,3 |
| Proteinhydrolysat | | 0,3 |
| $Na_2HPO_4$ | | 0,4 |
| $H_3PO_4$ | | 0,7 |
| Stabilisatoren, Parfümöl | | q.s. |
| Wasser | ad | 100,0 |

## Patentansprüche

1. Mittel zur dauerhaften Verformung von menschlichen Haaren, enthaltend mindestens ein Reduktionsmittel und mindestens ein $C_3$-$C_6$-Alkandiol bzw. dessen Ether, gekennzeichnet durch einen Gehalt an 0,5 bis 10 Gew.-%, berechnet auf die Gesamtzusammensetzung, Ammoniumcarbamat.

2. Mittel nach Anspruch 1, gekennzeichnet durch einen Gehalt an 2 bis 8 Gew.-%, berechnet auf die Gesamtzusammensetzung, Ammoniumcarbamat.

3. Mittel nach Anspruch 1 oder 2, dadurch gekennzeichnet, daß es 0,5 bis 35 Gew.-%, berechnet auf die Gesamtzusammensetzung, eines $C_3$-$C_6$-Alkandiols und/oder dessen Monoether enthält.

4. Mittel nach Anspruch 3, dadurch gekennzeichnet, daß es 1 bis 15 Gew.-%, berechnet auf die Gesamtzusammensetzung, eines $C_3$-$C_6$-Alkandiols und/oder dessen Monoether enthält.

5. Mittel nach einem der vorhergehenden Ansprüche, dadurch gekennzeichnet, daß es 1,2-Propandiol, 1-Methoxypropanol (-2) und/oder ein Butandiol enthält.

6. Mittel nach einem der vorhergehenden Ansprüche, dadurch gekennzeichnet, daß es 1 bis 30 Gew.-%, berechnet auf die Gesamtzusammensetzung, 4-Methyl-1,3-dioxolan-2-on enthält.

7. Verwendung von Ammoniumcarbamat als Alkalisierungsmittel in Reduktionsmittel-Zusammensetzungen zur dauerhaften Verformung von menschlichen Haaren.

## Claims

1. Composition for permanent waving of human hair, containing at least a reducing component and at least one $C_3$-$C_6$-alkanediol or an ether thereof, characterized in that it contains 0.5 to 10% by weight ammonium carbamate, calculated to the total composition.

2. Composition according to claim 1, characterized in that it contains 2 to 8% by weight ammonium carbamate, calculated to the total composition.

3. Composition according to claim 1 or 2, characterized in that it contains 0.5 to 35% by weight of a $C_3$-$C_6$-alkanediol and (or) a monoether thereof, calculated to the total composition.

4. Composition according to claim 3, characterized in that it contains 1 to 15% by weight of a $C_3$-$C_6$-alkanediol and (or) a monoether thereof, calculated to the total composition.

5. Composition according to one of the preceding claims, characterized in that it contains 1,2-propanediol, 1-methoxpropanol(-2) and (or) a butanediol.

6. Composition according to one of the preceding claims, characterized in that it contains 1 to 30% by weight 4-methyl-1,3-dioxolane-2-one, calculated to the total composition.

7. Use of ammonium carbamate as alkalinizing compound in reducing compositions for permanent waving of human hair.

## Revendications

1. Composition pour la déformation permanente des cheveux humains contenant au moins un agent réducteur et au moins un $C_3$-$C_6$-alkanediol ou son éther, caracterisée en ce qu'elle contient 0,5 à 10% en poids, calculé à partir de la composition finale, de carbamate d'ammonium.

2. Composition selon la revendication 1, caracterisée en ce qu'elle contient 2 à 8% en poids de carbamate d'ammonium, calculé à partir de la composition finale.

3. Compositions selon les revendications 1 ou 2, caracterisée en ce quelle contient 0,5 à 35% en poids d'un $C_3$-$C_6$-alkanediol et (ou) d'un monoéther de cela, calculé à partir de la composition finale.

4. Composition selon revendication 3, caracterisée en ce qu'elle contient 1 à 15 % en poids d'un $C_3$-$C_6$-alkanediol et (ou) d'un monoéther de cela, calculé à partir de la composition finale.

5. Composition selon l'une quelconque des revendications précédentes, caracterisée en ce qu'elle contient 1,2-propanediol, 1-méthoxypropanol(-2), et (ou) d'un butanediol.

6. Composition selon l'une quelconque des revendications précédentes, caracterisée en ce qu'elle contient 1 a 30% en poids de 4-méthyl-1,3-dioxolane 2-one, calculé à partir de la composition finale.

7. Utilisation de carbamate d'ammonium comme agent d'alcalinisation en compositions reductrices pour la dèformation permanente des cheveux humains.